# EUROPÄISCHE PATENTSCHRIFT

(11) **EP 0 616 796 B1**
(45) Veröffentlichungstag und Bekanntmachung des Hinweises auf die Patenterteilung: **27.05.1998**
(21) Anmeldenummer: 93810207.6
(22) Anmeldetag: 23.03.1993
(51) Int. Cl.: A61F 2/46, A61F 2/30, F16B 2/08, B65D 63/10

(54) **Bandförmiges Dichtelement**
Striplike sealing element
Bande d'étanchéité

(43) Veröffentlichungstag der Anmeldung: 28.09.1994
(73) Patentinhaber: Sulzer Orthopädie AG, 6340 Baar (CH)
(72) Erfinder: Spotorno, Lorenzo, Prof. Dr.-med., I-17024 Finale Ligure (IT); Frick, Willi, Dr., CH-3084 Wabern (CH); Willi, Roland, CH-8413 Neftenbach (CH)
(74) Vertreter: Hammer, Bruno, Dr.

(56) Entgegenhaltungen:
- EP-A- 0 073 604
- EP-A- 0 315 283
- EP-A- 0 516 569
- WO-A-90/02284
- FR-A- 2 662 931
- US-A- 4 997 448
- US-H- 968

## Beschreibung

Die Erfindung bezieht sich auf ein bandförmiges Dichtelement gemäss dem Oberbegriff von Anspruch 1.

Prothesen, beispielsweise Hüftprothesen, weisen einen Prothesenschaft auf, der mittels Knochenzement im Knochen verankert wird. Dazu wird ein Knochenhohlraum in den Knochen gebohrt, dieser teilweise mit Knochenzement gefüllt, und anschliessend der Prothesenschaft in den Knochenhohlraum eingeführt. Während des Einführens wird der Knochenzement verdrängt. Der Zement sucht sich einen Weg ins Freie und wird an der Einführstelle des Prothesenschaftes, im Spalt zwischen Knochenhohlraum und Prothesenschaft, nach aussen gepresst. Das Hinausfliessen des Knochenzementes wird üblicherweise behindert, um den Knochenzement unter einem gewissen Druck zu halten, damit der Knochenzement auch in kleine Poren und Ritzen dringt. Um eine Dichtungsfunktion zu erzielen ist bekannt, eine weiche Platte aus Kunststoff auf die Knochenöffnung zu legen und diese während dem Einführen des Prothesenschaftes zu durchstossen. Diese Art der Dichtung weist verschiedene Nachteile auf. Die Dichtungsfunktion hängt davon ab, wie die weiche Platte durchstossen wird. Die Dichtungsfunktion und somit der Druck im Knochenzement ist nicht reproduzierbar. Zudem wird das Einführen des Prothesenschaftes in den Knochenhohlraum behindert, da unmittelbar vor dem Einführen die Dichtung zu durchstossen ist.

Aus der FR-A-2662931 ist ein Dichtelement zum Abdichten des während dem Einführen eines Prothesenschaftes in den Hohlraum eines Knochens sich zwischen der Öffnung des Hohlraums und dem Prothesenschaft ergebenden Spaltes bekannt. Dieses Dichtelement ist als ein ebenes Teil mit einer entsprechend dem Querschnitt des Prothesenschaftes angepasst verlaufenden Ausnehmung ausgestaltet. Ein Nachteil dieses Dichtelementes ist darin zu sehen, dass während dem Einführen des Prothesenschaftes dessen Querschnitt an der Stelle der Öffnung des Hohlraumes variiert, und deshalb das an der Öffnung des Hohlraumes aufliegende Dichtelement erst dann vollständig am Prothesenschaft anliegt, wenn dieser beinahe vollständig in den Hohlraum eingeführt ist. Die Dichtungsfunktion und somit der Druck im Knochenzement ist daher nicht reproduzierbar. Zudem wird das Einführen des Prothesenschaftes in den Knochenhohlraum behindert, da während dem Einführen ständig auf die richtige Lage des Dichtelementes zu achten ist.

Der Erfindung liegt die Aufgabe zugrunde, diese Nachteile zu beheben.

Erfindungsgemäss wird diese Aufgabe gelöst gemäss den kennzeichnenden Merkmalen von Anspruch 1. Die Unteransprüche beziehen sich auf weitere, vorteilhafte Ausführungsformen der erfindungsgemässen Vorrichtung.

Die Vorteile der Erfindung sind darin zu sehen, dass das Dichtelement eine durch die geometrische Anordnung vorgegebene, reproduzierbare Dichtungsfunktion ausübt, wobei die Dichtungsfunktion durch eine entsprechende Gestaltung des Dichtelementes in weiten Grenzen variierbar ist. Durch die Materialwahl des Dichtelementes, von elastisch, weichem Material bis zu stabilem, harten Material, kann die Dichtungsfunktion beeinflusst werden. Weiter ist die Zugkraft, mit der das Dichtelement den Prothesenschaft umspannt, durch die Klemm- oder Schliessvorrichtung in einem weiten Bereich variierbar. Zudem können im Dichtelement Ausflussöffnungen für den Knochenzement vorgesehen werden, was ein kontrolliertes Abfliessen ermöglicht. Ein weiterer Vorteil ist darin zu sehen, dass das Dichtelement bereits vor dem Einführen des Prothesenschaftes an diesen anlegbar ist, so dass während des Einführens der Handlungsschritt entfällt, eine Kunststoffplatte zu durchstossen. Ein weiterer Vorteil ist darin zu sehen, dass die Dichtungsfunktion noch während des Einführens des Prothesenschaftes veränderbar ist, indem die Zugkraft im Dichtelement durch das Anziehen des Endabschnittes erhöht wird.

Im folgenden wird die Erfindung anhand von Ausführungsbeispielen beschrieben. Es zeigen:
- Fig. 1a: Ein bandförmiges Dichtelement, das einen Prothesenschaft umschlingt;
- Fig. 1b: einen Endabschnitt des Dichtelementes;
- Fig. 1c: eine Frontansicht der Schliessvorrichtung des Dichtelementes;
- Fig. 1d: einen schnitt (D-D) entlang des Endabschnittes zur Darstellung der Zahnung;
- Fig. 1e: einen schnitt durch die Schliessvorrichtung des Dichtelementes;
- Fig. 2a: einen schnitt durch einen Oberschenkelknochen mit eingeführter Prothese;
- Fig. 2b: ein Detail einer möglichen Oberflächenstruktur eines Prothesenschaftes;
- Fig. 3: ein weiteres, bandförmiges Dichtelement, das einen Prothesenschaft umschlingt;
- Fig. 3a-c: einen Schnitt (E-E) durch verschiedene Ausführungsformen des Umschlingungsabschnittes.

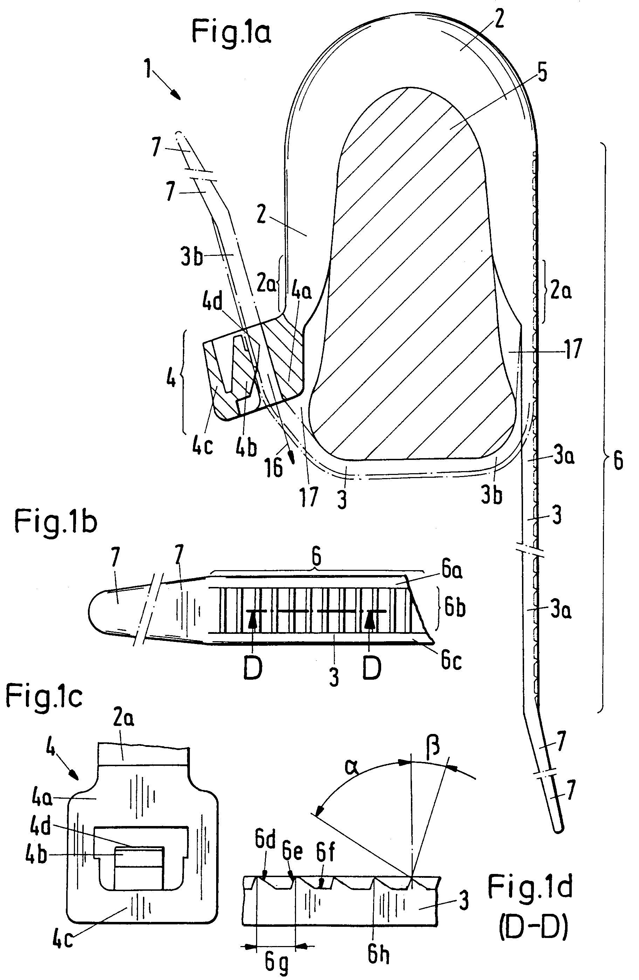

Fig. 1a zeigt ein bandförmiges Dichtelement 1, das einen Prothesenschaft 5 umschlingt. Das bandförmige Dichtelement 1 besteht aus einer Klemm- oder Schliessvorrichtung 4 und einem daran anschliessenden Umschlingungsabschnitt 2, der in einem Endabschnitt 3 ausläuft. Die Klemm- oder Schliessvorrichtung 4 hat die Aufgabe, den Endabschnitt 3 unter einer Zugspannung 16 zu halten, so dass das gesamte bandförmige Dichtelement 1 unter einer Vorspannung den Prothesenschaft 5 umschlingt. Das Halten eines bandförmigen Elementes unter Vorspannung kann natürlich mit verschiedensten Ausführungsformen gelöst werden. Die dargestellte Klemm- oder Schliessvorrichtung 4 ist somit nur als eine Ausführungsform aus einer Vielzahl von möglichen Ausführungsformen zu verstehen. Die dargestellte Klemmvorrichtung 4 besteht aus einer Backe 4a, einer weiteren Backe 4c, sowie einer federnden Zunge 4b, die einen auf den Endabschnitt 3 wirkenden Zahn 4d aufweist. Der Endabschnitt 3 ist in zwei möglichen Lagen dargestellt, als Endabschnitt 3a in losem Zustand und als Endabschnitt 3b in befestigtem Zustand, in dem der Endabschnitt 3b durch die Klemmvorrichtung 4 geschlauft von dieser unter Vorspannung 16 gehalten wird. Der Endabschnitt 3 läuft in eine Spitze 7 aus, die das Einfädeln des Endabschnitts 3 in die Klemmvorrichtung 4 zwischen Backe 4a und federnder Zunge 4b erleichtert. Der Umschlingungsabschnitt 2 ist radial zur Umschlingungsrichtung des Dichtelementes 1 relativ breit ausgestaltet, um den Spalt 8 zwischen Knochen 9 und Prothesenschaft 5 auszufüllen. Der Endabschnitt 3 dagegen ist radial zur Umschlingung relativ schmal ausgeführt, derart, dass er in die Klemmvorrichtung 4 einführbar ist. Somit ergibt sich ein Uebergangsbereich 2a, der vom relativ breiten Umschlingungsabschnitt 2 zum schmaleren Endabschnitt 3 überführt, oder der vom relativ breiten Umschlingungsabschnitt 2 zur Klemmvorrichtung 4 überführt.

Im vorliegenden Ausführungsbeispiel weist der Endabschnitt 3 sowie der daran anschliessende Uebergangsbereich 2a an der Aussenseite einen Zahnungsbereich 6 auf, der eine fortlaufende Zahnung aufweist. Das bandförmige Dichtelement 1 weist Bereiche 17 auf, in denen das Dichtelement 1 nicht am Prothesenschaft 5 anliegt. Somit ergibt sich für Bereiche 17 keine dichtende Wirkung. Die Grösse der offenen Bereiche 17 kann entsprechend der Gestaltung des Ueberganges 2a zwischen dem Umschlingungsabschnitt 2 und dem Endabschnitt 3 resp. der Klemmvorrichtung 4 gestaltet werden. Natürlich ist es auch möglich, im Umschlingungsabschnitt 2 offene Bereiche 17 vorzusehen, indem im Umschlingungsabschnitt derart Ausnehmungen vorgesehen sind, dass sich zwischen Umschlingungsabschnitt 2 und Prothesenschaft 5 nicht anliegende Bereiche ergeben.

Fig. 1b zeigt eine Aufsicht auf einen Endabschnitt 3 eines Dichtungselementes 1. Der Endabschnitt 3 weist im Bereich 6 eine Zahnung auf. Der Zahnungsbereich 6 weist auf jeder Seite eine Randbegrenzung 6a, 6c auf, die auch zur Führung des Endabschnittes 3 in der Klemmvorrichtung 4 dient. Zwischen den Randbegrenzungen 6a, 6c befindet sich der Verzahnungsbereich 6b, der fortlaufend Zähne aufweist. Weiter ist die Spitze 7 des Endabschnittes 3 dargestellt.

Eine Schnittzeichnung entlang D-D ist in Fig. 1d dargestellt. Daraus werden die einzelnen Zähne 6a des Zahnungsbereiche 6 ersichtlich. Die Zähne sind in einem üblicherweise regelmässigen Abstand 6g angeordnet, wobei die Zähne eine steigende Zahnflanke 6e und eine fallende Zahnflanke 6d aufweisen, wobei der Neigungswinkel der Zahnflanken bezüglich einer vertikal zum Endabschnitt 3 verlaufenden Ausrichtung einen Steigungswinkel α resp. einen Steigungswinkel β aufweisen.

Fig. 1c zeigt eine Frontansicht der Klemmvorrichtung 4, die mit dem Uebergangsbereich 2a des Umschlingungsabschnittes 2 verbunden ist. Die Klemmvorrichtung 4 weist eine Backe 4a auf sowie auf der entgegengesetzten Seite eine Backe 4c. An der Backe 4c ist eine federnde Zunge 4b und an deren Spitze ein Zahn 4d angeordnet.

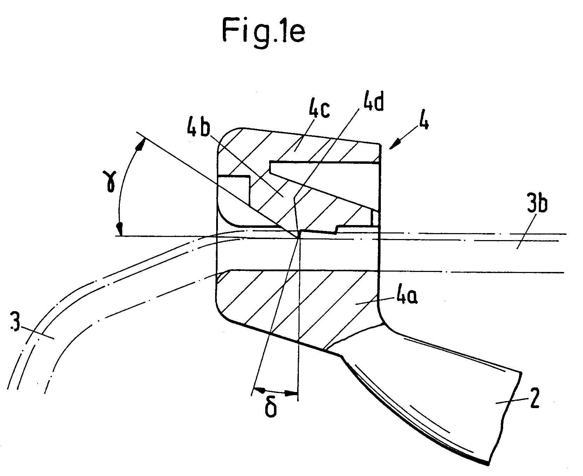

Fig. 1e zeigt einen Querschnitt durch die Klemmvorrichtung 4. Die haltende Wirkung wird durch die Interaktion des Zahnungsbereichs 6 des Endabschnittes 3 mit dem Zahn 4d der Klemmvorrichtung 4 erzielt. Die Wirkung des Zahnes 4d ist insbesondere durch die beiden Steigungswinkel Γ und δ der Zahnung bestimmt. Die Wirkung der Klemmvorrichtung 4 auf den Endabschnitt 3 ist durch die Federkraft der Zunge 4b sowie durch die Gestaltung des Zahnes 4d sowie durch die Gestaltung der Zähne 6h des Endabschnittes 3 bestimmt, insbesondere durch die gegenseitige Abstimmung der Winkel α, β, Γ und δ. Die Wahl der Winkel bestimmt, ob die Verbindung zwischen der Klemmvorrichtung 4 und dem Endabschnitt 3 z.B. nicht mehr lösbar ist oder ob die Verbindung wieder lösbar ist. Im Falle einer lösbaren Verbindung beeinflussen die Winkelverhältnisse die Zugkraft 16, die überschritten werden muss, um den eingespannten Teil des Endabschnitts 3 zu verlängern. Die Bewegung des Endabschnitts 3 in Richtung der Zugkraft 16 erfolgt, entsprechend dem Abstand 6g der Zahnung, ruckartig. Die Zugkraft, die erforderlich ist, um den Endabschnitt 3 aus der Klemmvorrichtung 4 zu ziehen, ist entsprechend den Winkelverhältnissen in weiten Grenzen variierbar. Es sind Dichtelemente 1 mit unterschiedlicher, maximal haltbarer Zugkraft 16 herstellbar.

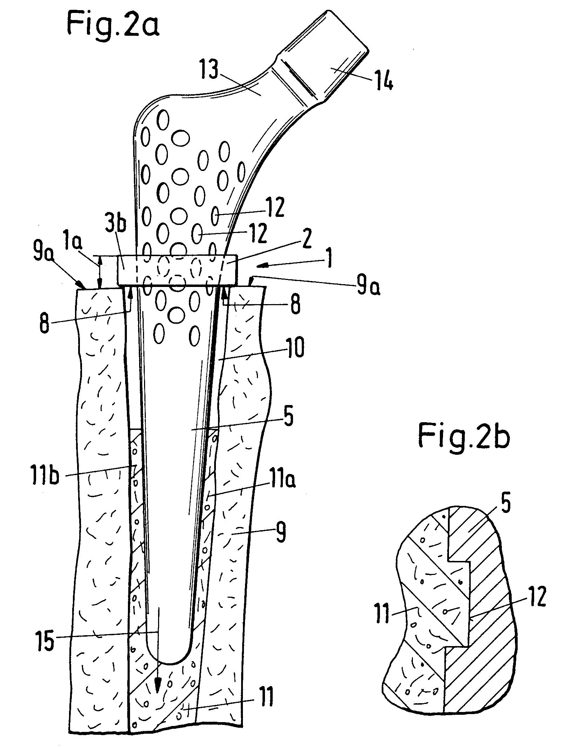

Die Fig. 2a zeigt einen Schnitt durch einen Oberschenkelknochen 9, in den eine Prothese mit Prothesenschaft 5, Prothesenhals 13 und konischem Zapfen 14 in Einführungsrichtung 15 eingeführt wird. Der Knochen 9 ist derart bearbeitet, dass er einen genügend grossen Knochenhohlraum 10 aufweist, der üblicherweise durch eine entsprechende Innenbohrung erzeugt wird, sowie eine üblicherweise definierte Schnittstelle oder Auflagefläche 9a. Nach dem Bohren des Knochenhohlraumes 10 wird in diesen ein Knochenzement 11 eingeführt, der während des Einführens des Prothesenschaftes 5 in Einführungsrichtung 15 entlang dem Spalt zwischen Prothesenschaft 5 und Knochen 9 als aufsteigender Knochenzement 11a, 11b gegen den Spalt 8 hin fliesst. Das Dichtelement 1 mit Umschlingungsabschnitt 2 wurde vorgängig dem Einführen des Prothesenschaftes 5 unter Vorspannung um den Prothesenschaft 5 gelegt und kommt während des Einführens des Prothesenschaftes in den Knochenhohlraum 10 irgendwann auf die Schnittstelle 9a zu liegen. Somit wird der Spalt 8 zwischen Knochen 9 und Prothesenschaft 5 durch das Dichtelement 1 teilweise oder vollständig abgedichtet. Der aufsteigende Knochenzement 11a, 11b wird somit durch das Dichtelement 1 zurückgehalten, und durch den sich erhöhenden Druck im Knochenzement füllt dieser auch feine Poren im Knochen 9 sowie, wie in Fig. 2b dargestellt, Vertiefungen 12 in der Oberfläche des Prothesenschaftes 5 vollständig mit Knochenzement 11 aus. Dabei wird die Breite 1a des Dichtelementes 1 vorzugsweise derart gewählt, dass die Breite 1a die Dimension einer Vertiefung 12 bezüglich Einführungsrichtung 15 übersteigt. Um eine Beschädigung des Knochens 9 zu vermeiden, darf der aufgebaute Druck im Knochenzement 11, 11a, 11b nicht zu hoch ausfallen. Daher sind z.B. offene Bereiche 17 zwischen Dichtelement 1 und Prothesenschaft 5 vorgesehen, wie dies in Fig. 1a dargestellt ist, um einen kontrollierten Austritt des Knochenzementes 11 zu gewährleisten. Wird ein bandförmiges Dichtelement 1 derart ausgestaltet, dass sich zwischen Prothesenschaft 5 und Dichtelement 1 keine offenen Bereiche 17 ergeben, so entsteht durch den sich aufbauenden Druck im Knochenzement 11 ein Spalt zwischen Dichtelement 1 und der Schnittstelle 9a, durch den der Knochenzement 11 ausfliesst. Die resultierende Spaltbreite zwischen Dichtelement 1 und Schnittstelle 9a hängt unter anderem ab von der Zugspannung 16, mit der der Endabschnitt 3 durch die Klemmvorrichtung 4 gehalten wird. Wie erwähnt, ist die maximale Zugspannung 16 durch die Gestaltung der Zähne 6h und durch die Gestaltung der Klemmrichtung 4 in weiten Grenzen variierbar, somit ist auch der Druck des Knochenzementes 11 durch eine entsprechende Wahl eines Dichtungselementes 1 beeinflussbar.

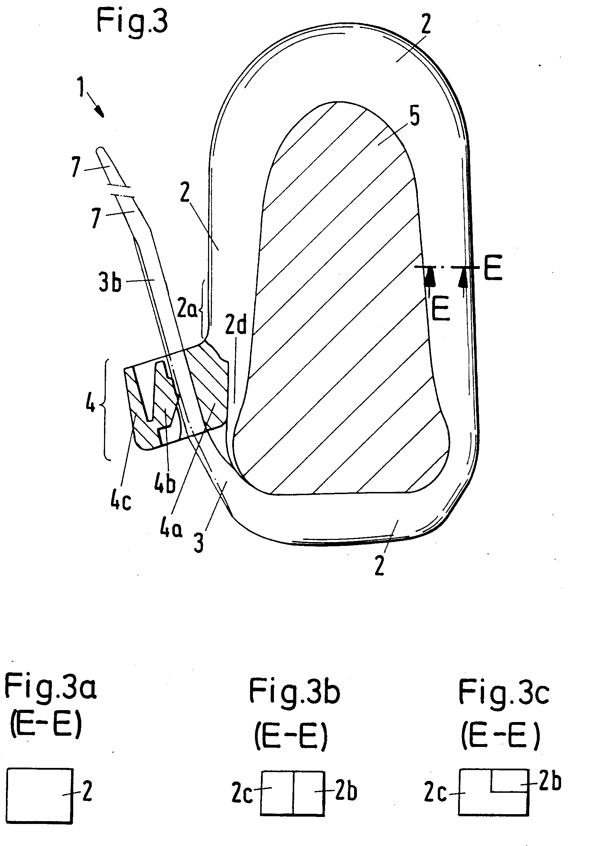

Fig. 3 zeigt ein weiteres Ausführungsbeispiel eines bandförmigen Dichtelementes 1. Dieses Ausführungsbeispiel weist keine offenen Bereiche 17 zwischen Dichtelement 1 und Prothesenschaft 5 auf und erlaubt somit eine vollständige Dichtung zwischen Dichtelement 1 und Prothesenschaft 5. Der Umschlingungsabschnitt 2 umschlingt im vorliegenden Ausführungsbeispiel den Prothesenschaft 5 vollständig und läuft erst in der Nähe der Klemm- oder Schliesvorrichtung 4 in einen Endabschnitt 3 aus, der durch die Klemm- oder Schliessvorrichtung 4 unter Vorspannung gehalten ist. Zur besseren Dichtung weist der Umschlingungsabschnitt 2 im Bereich der Klemmvorrichtung 4 einen Lappenfortsatz 2d auf, der einen möglichen Spalt zwischen Klemmvorrichtung 4 und Prothesenschaft 5 sowie evtl. einen Spalt zwischen Endabschnitt 3 und Prothesenschaft 5 dichtet. Ein Schnitt durch den Umschlingungsabschnitt 2 entlang der Linie E-E ist in den Fig. 3a bis 3c dargestellt. Das bandförmige Dichtelement 1, insbesondere der Umschlingungsabschnitt 2 kann aus Materialien unterschiedlicher Eigenschaften oder auch mehrschichtig aufgebaut sein.

Fig. 3a zeigt einen einschichtigen Aufbau des Umschlingungsabschnittes 2. Um gute Dichtungseigenschaften zwischen dem Umschlingungsabschnitt 2 und dem Prothesenschaft 5 zu erreichen, weist der Umschlingungsabschnitt 2 vorteilhafterweise elastische Eigenschaften auf, so dass er sich dem Prothesenschaft 5 überall anschmiegt.

Fig. 3b zeigt einen zweischichtigen Aufbau des Umschlingungsabschnittes 2. Der innere, am Prothesenschaft 5 anliegende Umschlingungsabschnitt 2c wird dabei vorteilhafterweise elastisch ausgeführt, wogegen der äussere Umschlingungsabschnitt 2b weniger elastische, stabile Eigenschaften aufweist. So ist es auch möglich, dass der Umschlingungsabschnitt 2b und der Endabschnitt 3 aus einem Stück gefertigt sind, so dass der innere, elastische Umschlingungsabschnitt 2c von einem äusseren, weniger elastischen Umschlingungsabschnitt 2b gehalten wird, der mit der Klemmvorrichtung 4 verbindem, den Prothesenschaft 5 umschlingt und in den Endabschnitt 3 ausläuft.

Fig. 3c zeigt ein weiteres Ausführungsbeispiel eines Umschlingungsabschnittes 2, wobei der innere, elastische Umschlingungsabschnitt 2c L-förmig ausgebildet ist, derart, dass der elastische Bereich am Prothesenschaft 5 anliegt sowie auf die Schnittstelle 9a des Knochens 9 zu liegen kommt. Der äussere, weniger oder völlig unelastische Umschlingungsabschnitt 2b gewährleistet die Umfassungsspannung um den Prothesenschaft 5, hervorgerufen durch die Klemmvorrichtung 4.

Die Klemmvorrichtung 4 kann natürlich auch derart ausgestaltet sein, dass der Endabschnitt 3 unnachgiebig mit der Klemmvorrichtung 4 verbunden ist. In diesem Falle muss der Umschlingungsabschnitt 2 und/oder der Endabschnitt 3 elastische Eigenschaften aufweisen, so dass sich der Umfang des eingespannten Dichtelementes 1 entsprechend der Umfangszunahme des Prothesenschaftes 5 diesem angleichen kann.

## Patentansprüche

1. Dichtelement (1) zum Abdichten des während dem Einführen eines Prothesenschaftes (5) in den Hohlraum eines Knochens sich zwischen der Öffnung des Hohlraums und dem Prothesenschaft (5) ergebenden Spaltes, dadurch gekennzeichnet, dass das Dichtelement (1) bandförmig ausgestaltet ist, bestehend aus einer Klemm- oder Schliessvorrichtung (4), und einem daran anschliessenden Umschlingungsabschnitt (2), der in einen Endabschnitt (3) ausläuft, wobei der Endabschnitt (3) in die Klemm- oder Schliessvorrichtung (4) einführbar ist, und wobei das Dichtelement (1) in einer den Prothesenschaft (5) umspannenden Anordnung an diesen anlegbar ist.

2. Dichtelement (1) nach Anspruch 1, dadurch gekennzeichnet, dass der Endabschnitt (3) und die Klemm- oder Schliessvorrichtung (4) derart gegenseitig wirkend ausgestaltet sind, dass der Endabschnitt (3) unnachgiebig in der Klemm- oder Schliessvorrichtung (4) festgehalten ist.

3. Dichtelement (1) nach Anspruch 1, dadurch gekennzeichnet, dass der Endabschnitt (3) und die Klemm- oder Schliessvorrichtung (4) derart gegenseitig wirkend ausgestaltet sind, dass der Endabschnitt (3) in Abhängigkeit der ausgeübten Zugkraft nachgiebig in der Klemm- oder Schliessvorrichtung (4) gehalten ist.

4. Dichtelement (1) nach Anspruch 1, dadurch gekennzeichnet, dass der Endabschnitt (3) und die Klemm- oder Schliessvorrichtung (4) derart gegenseitig wirkend ausgestaltet sind, dass der Endabschnitt (3) nachgiebig in der Klemm- oder Schliessvorrichtung (4) gehalten ist, und die vom Endabschnitt (3) auf die Klemm- oder Schliessvorrichtung (4) bewirkte Zugkraft ungefähr konstant bleibt.

5. Dichtelement (1) nach Anspruch 1, dadurch gekennzeichnet, dass der Endabschnitt (3) und die Klemm- oder Schliessvorrichtung (4) derart gegenseitig wirkend ausgestaltet sind, dass der Endabschnitt (3) nachgiebig in der Klemm- oder Schliessvorrichtung (4) gehalten ist, und die Klemm- oder Schliessvorrichtung (4) beim Ueberschreiten einer bestimmten Zugkraft schrittweise eine Teillänge des Endabschnittes (3) freigibt.

6. Dichtelement (1) nach einem der Ansprüche 1 bis 5, dadurch gekennzeichnet, dass der Umschlingungsabschnitt (2) unelastisch ausgestaltet ist.

7. Dichtelement (1) nach einem der Ansprüche 1 bis 5, dadurch gekennzeichnet, dass der Umschlingungsabschnitt (2) elastisch ausgestaltet ist.

8. Dichtelement (1) nach einem der Ansprüche 1 bis 5, dadurch gekennzeichnet, dass der Umschlingungsabschnitt (2) längs seiner Verlaufsrichtung zweischichtig ausgestaltet ist und aus einer elastischen Schicht (2c) und einer weniger elastischen Schicht (2b) besteht.

9. Dichtelement (1) nach Anspruch 8, dadurch gekennzeichnet, dass der weniger elastische Umschlingungsabschnitt (2b) in den Endabschnitt (3) ausläuft, welcher mit der Klemm- oder Schliessvorrichtung (4) (3) in Wirkverbindung zu gelangen bestimmt ist.

10. Dichtelement (1) nach einem der Ansprüche 3 bis 9, dadurch gekennzeichnet, dass die Klemm- oder Schliessvorrichtung (4) eine federnde Zunge (4b) mit einem Zahn (4d) aufweist, und dass das Endstück (3) einen Zahnungsbereich (6) mit in Längsrichtung beabstandeten Zähnen (6h) aufweist, wobei die Steigungswinkel der Zähne (6h) als auch des Zahnes (4d) zusammen mit der Federkraft der Zunge (4b) die haltbare Zugkraft bestimmen.

## Claims

1. Sealing element (1) for the sealing of the gap resulting during the introduction of a shaft (5) of a prosthesis into the hollow cavity of a bone between the opening of the hollow cavity and the shaft (5) of the prosthesis, characterized in that the sealing element (1) is of band-like form, comprises a clamping or closure device (4) and a wrapping section (2) adjoining it, which runs out into an end section (3), with the end section (3) being introducible into the clamping or closure device (4), and wherein the sealing element (1) can be brought into contact with the shaft (5) of the prosthesis in an arrangement surrounding it.

2. Sealing element (1) in accordance with claim 1, characterized in that the end section (3) and the clamping or closure device (4) are designed to mutually cooperate in such a way that the end section (3) is non-yieldably held in the clamping or closure device (4).

3. Sealing element (1) in accordance with claim 1, characterized in that the end section (3) and the clamping or closure device (4) are designed to mutually cooperate in such a way that the end section (3) is yieldably held in the clamping or closure device (4) in dependence on the tension force that is exerted.

4. Sealing element (1) in accordance with claim 1, characterized in that the end section (3) and the clamping or closure device (4) are designed to mutually cooperate in such a way that the end section (3) is yieldably held in the clamping or closure device (4) and the tension force exerted by the end section (3) onto the clamping or closure device (4) remains approximately constant.

5. Sealing element (1) in accordance with claim 1, characterized in that the end section (3) and the clamping or closure device (4) are designed to mutually cooperate in such a way that the end section (3) is yieldably held in the clamping or closure device (4) and the clamping or closure device (4) releases a part length of the end section (3) stepwise when a specific tension force is exceeded.

6. Sealing element (1) in accordance with one of the claims 1 to 5, characterized in that the wrapping section (2) is made inelastic.

7. Sealing element (1) in accordance with one of the claims 1 to 5, characterized in that the wrapping section (2) is made elastic.

8. Sealing element (1) in accordance with one of the claims 1 to 5, characterized in that the wrapping section (2) is made with two layers along its direction of extent and consists of an elastic layer (2c) and a less elastic layer (2b).

9. Sealing element (1) in accordance with claim 8, characterized in that the less elastic wrapping section (2b) runs out into the end section (3), which is intended to enter into co-operating connection with the clamping or closure device (4) (3).

10. Sealing element (1) in accordance with one of the claims 3 to 9, characterized in that the clamping or closure device (4) has a resilient tongue (4b) with a tooth (4d) and in that the end piece (3) has a toothed region (6) with teeth (6h) spaced apart in the longitudinal direction, with the flank angle of the teeth (6h) and also of the tooth (4d) together with the spring force of the tongue (4b) jointly determining the maintainable tension force.

## Revendications

1. Elément d'étanchéité (1) pour rendre étanche la fente produite pendant l'insertion d'un corps de prothèse (5) dans l'espace creux d'un os entre l'ouverture de l'espace creux et le corps de prothèse (5), caractérisé en ce que l'élément d'étanchéité (1) est réalisé en forme de bande, constitué d'un dispositif de serrage ou de fermeture (4) et d'un tronçon d'enroulement (2) faisant suite à celui-ci qui se termine par un tronçon d'extrémité (3), le tronçon d'extrémité (3) pouvant être inséré dans le dispositif de serrage ou de fermeture (4) et où l'élément d'étanchéité (1), suivant une disposition enserrant le corps de prothèse (5), peut être appliqué à celui-ci.

2. Elément d'étanchéité (1) selon la revendication 1, caractérisé en ce que le tronçon d'extrémité (3) et le dispositif de serrage ou de fermeture (4) sont réalisés de façon à agir l'un contre l'autre de sorte que le tronçon d'extrémité (3) soit retenu d'une manière inflexible dans le dispositif de serrage ou de fermeture (4).

3. Elément d'étanchéité (1) selon la revendication 1, caractérisé en ce que le tronçon d'extrémité (3) et le dispositif de serrage ou de fermeture (4) sont réalisés pour agir l'un relativement à l'autre de façon que le tronçon d'extrémité (3), en fonction de la contrainte de traction exercée, soit tenu d'une manière flexible dans le dispositif de serrage ou de fermeture (4).

4. Elément d'étanchéité (1) selon la revendication 1, caractérisé en ce que le tronçon d'extrémité (3) et le dispositif de serrage ou de fermeture (4) sont réalisés de façon à agir l'un relativement à l'autre de manière que le tronçon d'extrémité (3) soit tenu d'une manière flexible dans le dispositif de serrage ou de fermeture (4) et que la contrainte de traction exercée par le tronçon d'extrémité (3) sur le dispositif de serrage ou de fermeture (4) reste approximativement constante.

5. Elément d'étanchéité (1) selon la revendication 1, caractérisé en ce que le tronçon d'extrémité (3) et le dispositif de serrage ou de fermeture (4) sont réalisés pour agir l'un relativement à l'autre de façon que le tronçon d'extrémité (3) soit tenu d'une manière flexible dans le dispositif de serrage ou de fermeture (4), et que le dispositif de serrage ou de fermeture (4), lors du dépassement d'une contrainte de traction déterminée, libère progressivement une longueur partielle du tronçon d'extrémité (3).

6. Elément d'étanchéité (1) selon l'une des revendications 1 à 5, caractérisé en ce que le tronçon d'enroulement (2) est réalisé de manière non élastique.

7. Elément d'étanchéité (1) selon l'une des revendications 1 à 5, caractérisé en ce que le tronçon d'enroulement (2) est réalisé de manière élastique.

8. Elément d'étanchéité (1) selon l'une des revendications 1 à 5, caractérisé en ce que le tronçon d'enroulement (2), le long de sa direction d'extension, est réalisé en deux couches et est constitué d'une couche élastique (2c) et d'une couche moins élastique (2b).

9. Elément d'étanchéité (1) selon la revendication 8, caractérisé en ce que le tronçon d'enroulement moins élastique (2b) se termine par le tronçon d'extrémité (3) qui est prévu pour entrer en liaison active avec le dispositif de serrage ou de fermeture (4).

10. Elément d'étanchéité (1) selon l'une des revendications 3 à 9, caractérisé en ce que le dispositif de serrage ou de fermeture (4) présente une languette élastique (4b) avec une dent (4d) et en ce que la pièce d'extrémité (3) présente une zone de denture (6) avec des dents (6h) espacées dans la direction longitudinale, l'angle d'inclinaison des dents (6h) ainsi que de la dent (4d) déterminant conjointement avec la force élastique de la languette (4b) la contrainte de traction soutenable.
